(19) Europäisches Patentamt
European Patent Office
Office européen des brevets

(11) Veröffentlichungsnummer: **0 057 872**
**A2**

(12) **EUROPÄISCHE PATENTANMELDUNG**

(21) Anmeldenummer: **82100609.5**

(22) Anmeldetag: **29.01.82**

(51) Int. Cl.³: **C 07 D 233/90**
A 01 N 43/50

(30) Priorität: **11.02.81 DE 3104759**

(43) Veröffentlichungstag der Anmeldung:
**18.08.82 Patentblatt 82/33**

(84) Benannte Vertragsstaaten:
**AT BE CH DE FR GB IT LI NL**

(71) Anmelder: **BAYER AG**
**Zentralbereich Patente, Marken und Lizenzen**
**D-5090 Leverkusen 1, Bayerwerk(DE)**

(72) Erfinder: **Beck, Gunther, Dr.**
**Am Mittelberg 19**
**D-5090 Leverkusen 1(DE)**

(72) Erfinder: **Baasner, Bernd, Dr.**
**Kalkstrasse 132**
**D-5090 Leverkusen 1(DE)**

(72) Erfinder: **Eue, Ludwig, Dr.**
**Paul-Klee-Strasse 36**
**D-5090 Leverkusen 1(DE)**

(72) Erfinder: **Schmidt, Robert, Dr.**
**Im Waldwinkel 110**
**D-5060 Bergisch-Gladbach 2(DE)**

(54) **Dihalogenierte Imidazolcarbonsäure-amide, Verfahren zu ihrer Herstellung und ihre Verwendung als Herbizide.**

(57) Dihalogenierte Imidazolcarbonsäure-amide der Formel

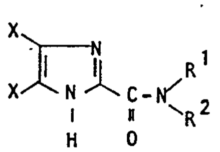

(1)

in welcher
x für Chlor oder Brom steht,
$R^1$ für Fluoralkyl oder Fluorchloralkyl steht und
$R^2$ für Wasserstoff oder Alkyl steht,
mehrere Verfahren zu deren Herstellung und ihre Verwendung als Herbizide.

EP 0 057 872 A2

Croydon Printing Company Ltd.

0057872

BAYER AKTIENGESELLSCHAFT 5090 Leverkusen, Bayerwerk
Zentralbereich
Patente, Marken und Lizenzen Dü/kl-c
Ib

# Dihalogenierte Imidazolcarbonsäure-amide, Verfahren zu ihrer Herstellung und ihre Verwendung als Herbizide

Die vorliegende Erfindung betrifft neue dihalogenierte Imidazolcarbonsäure-amide, mehrere Verfahren zu deren Herstellung sowie ihre Verwendung als Herbizide.

Es ist bereits bekannt geworden, daß bestimmte 4,5-Dichlorimidazol-2-carbonsäure-Derivate herbizide Eigenschaften besitzen (vgl. DE-A 2 610 527). So kann z.B. das 4,5-Dichlorimidazol-2-carbonsäure-tert.-butylamid zur Bekämpfung von Unkraut eingesetzt werden. Dieser Stoff ist jedoch nicht immer ausreichend wirksam und in seiner Selektivität nicht immer ganz befriedigend.

Es wurden nun neue dihalogenierte Imidazolcarbonsäure-amide der Formel

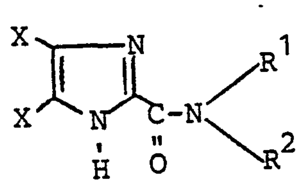

(I)

in welcher

X für Chlor oder Brom steht,

$R^1$ für Fluoralkyl oder Fluorchloralkyl steht und

$R^2$ für Wasserstoff oder Alkyl steht,

gefunden.

Weiterhin wurde gefunden, daß man dihalogenierte Imidazolcarbonsäure-amid der Formel (I) erhält, wenn man

a) das dimere Keten der Formel

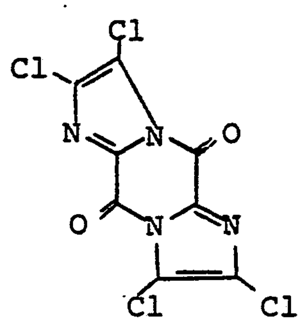

(II)

mit fluorierten Aminen der Formel

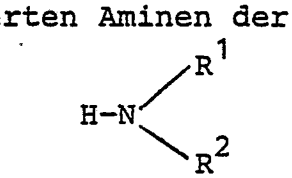

(III)

in welcher

$R^1$ und $R^2$ die oben angegebene Bedeutung haben,

gegebenenfalls in Gegenwart eines Verdünnungsmittels sowie gegebenenfalls in Gegenwart eines Katalysators, umsetzt,

oder

b) Imidazol-2-carbonsäure-amide der Formel

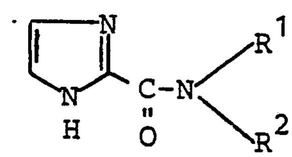

(IV)

in welcher

$R^1$ und $R^2$ die oben angegebene Bedeutung haben,

mit mindestens der zur Dihalogenierung stöchiometrisch erforderlichen Menge an Chlorierungs- oder Bromierungsmittel in Gegenwart eines unter den Reaktionsbedingungen inerten Verdünnungsmittels sowie gegebenenfalls in Gegenwart eines säurebindenden Mittels umsetzt,

oder

c) Perchlordiazafulven der Formel

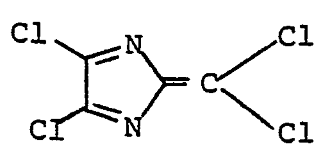

(V)

mit fluorierten Aminen der Formel

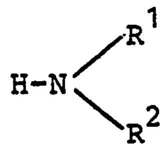

(III)

in welcher

$R^1$ und $R^2$ die oben angegebene Bedeutung haben,

in Gegenwart eines Verdünnungsmittels sowie gegebenenfalls in Gegenwart eines Säurebindemittels umsetzt und anschließend mit Wasser hydrolysiert.

Schließlich wurde gefunden, daß die neuen dihalogenierten Imidazolcarbonsäure-amide der Formel (I) starke herbizide, insbesondere auch selektiv-herbizide Eigenschaften besitzen.

Überrschenderweise zeigen die erfindungsgemäßen dihalogenierten Imidazolcarbonsäure-amide der Formel (I) bei sehr guter Unkrautwirkung insbesondere bessere Möglichkeiten zum Einsatz als selektive Unkrautbekämpfungsmittel in wichtigen Kulturen als das aus dem Stand der Technik bekannte 4,5-Dichlorimidazol-2-carbonsäure-tert.-butylamid, welches ein konstitutionell ähnlicher, hochaktiver Wirkstoff gleicher Wirkungsart ist.

Die erfindungsgemäßen Stoffe stellen smit eine wertvolle Bereicherung der herbiziden Mittel zur selektiven Unkrautbekämpfung dar.

Die erfindungsgemäßen dihalogenierten Imidazolcarbonsäure-amide sind durch die Formel (I) allgemein definiert. In dieser Formel steht X für Chlor oder Brom. $R^1$ steht vorzugsweise für geradkettiges oder verzweigtes Fluoralkyl mit 1 bis 8 Kohlenstoffatomen und 1 bis 9 Fluoratomen oder für geradkettiges oder verzweigtes Fluorchloralkyl mit 1 bis 8 Kohlenstoffatomen und bis zu 9 Fluor- und Chloratomen. $R^2$ steht vorzugsweise für Wasserstoff oder Alkyl mit 1 bis 6 Kohlenstoffatomen.

Besonders bevorzugt sind solche Verbindungen der Formel (I), in denen X für Chlor oder Brom steht, $R^1$ für geradkettiges oder verzweigtes Fluoralkyl mit 1 bis 6 Kohlenstoffatomen und 1 bis 7 Fluoratomen oder für geradkettiges oder verzweigtes Fluorchloralkyl mit 1 bis 6 Kohlenstoffatomen und bis zu 7 Fluor- und Chloratomen steht und $R^2$ für Wasserstoff oder Alkyl mit 1 bis 4 Kohlenstoffatomen steht.

Verwendet man bei der Variante (a) des erfindungsgemäßen Verfahrens das dimere Keten der Formel (II) und 1-Amino-3,3,3-trifluorpropan als Ausgangsstoffe, so kann der Verlauf der Umsetzung durch das folgende Formelschema wiedergegeben werden:

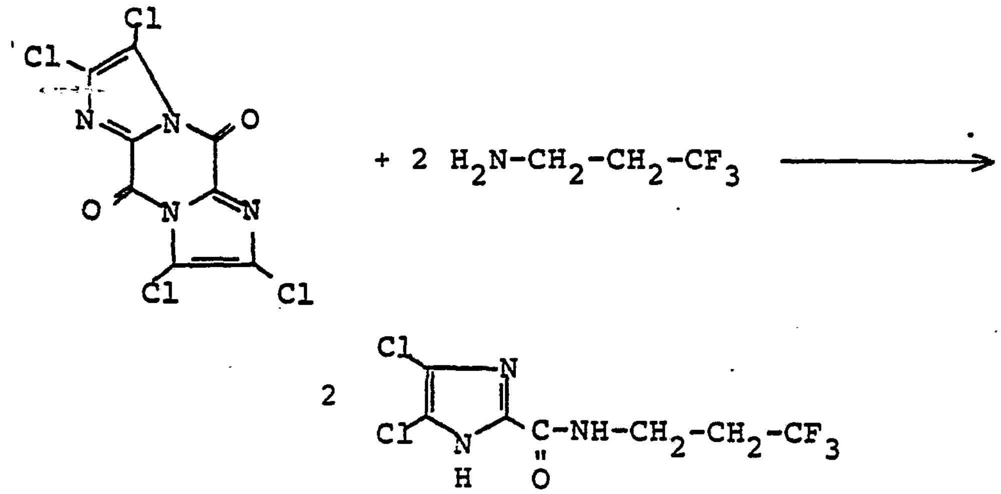

Verwendet man bei der Variante (b) des erfindungsgemäßen Verfahrens Imidazol-2-carbonsäure-N-(2,2,2-trifluorisopropyl)-amid und Brom als Ausgangsstoffe, so kann der Verlauf der Umsetzung durch das folgende Formelschema wiedergegeben werden:

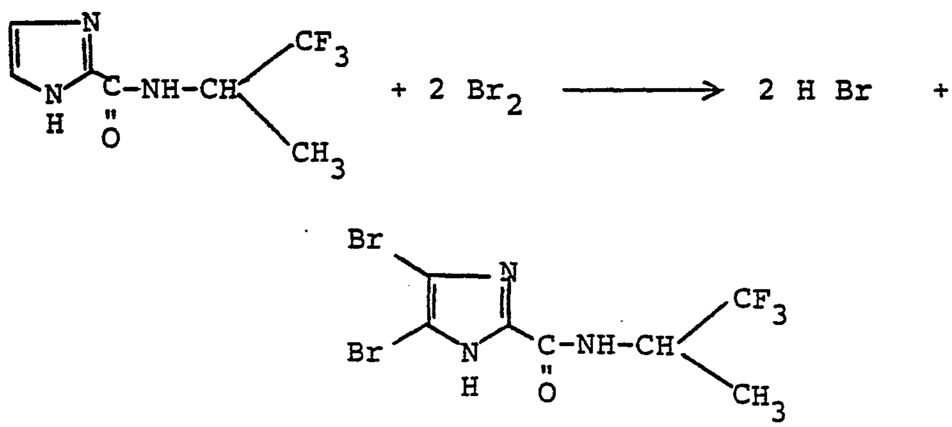

Verwendet man bei der Variante (c) des erfindungsgemäßen Verfahrens das Perchlordiazafulven der Formel (V) und 2-Amino-1,1,1-trifluorpropan als Ausgangsstoffe und Wasser als Hydrolysierungsmittel, so kann der Verlauf der Umsetzung durch das folgende Formelschema wiedergegeben werden:

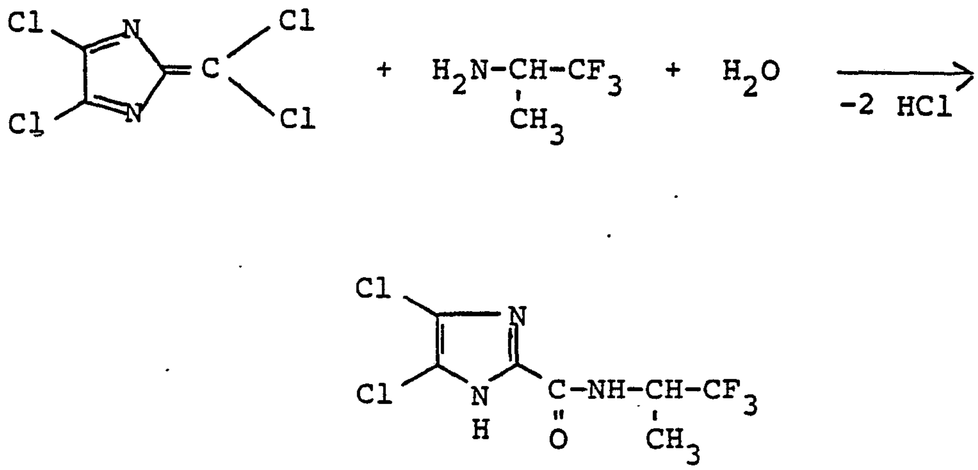


Das bei dem erfindungsgemäßen Verfahren (a) als Ausgangsstoff benötigte dimere Keten der Formel (II) ist bekannt (vgl. DE-A 26 34 053 ).

Die bei dem erfindungsgemäßen Verfahren (a) weiterhin als Ausgangsstoffe benötigten fluorierten Amine sind durch die Formel (III) allgemein definiert. In dieser Formel haben $R^1$ und $R^2$ vorzugsweise diejenigen Bedeutungen, die bereits im Zusammenhang mit der Beschreibung der erfindungsgemäßen Stoffe der Formel (I) vorzugsweise für diese Reste genannt wurden.

Die fluorierten Amine der Formel (III) sind teilweise bekannt (vgl. J. Org. Chem. 24, 1256 - 1259 (1959), J. Org. Chem. 27, 1406 - 1409 (1962), J. Med. Chem. 22, 1130 - 1133 (1979), Izv. Aka. Nauk. SSSR Ser. Khim. 1966, 1518 (engl.), US-A 3 908 012, US-PS 3 960 949 und DE-A 2 117 015). Die bisher noch nicht im einzelnen beschriebenen fluorierten Amine der Formel (III)

lassen sich nach im Prinzip bekannten Methoden herstellen. So erhält man z.B. diejenigen fluorierten Amine der Formel

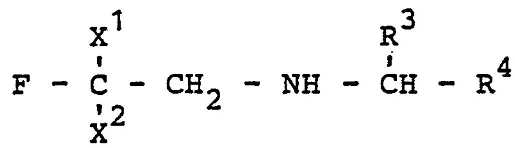

(VI)

in welcher

$X^1$ für Wasserstoff, Fluor oder Chlor steht,

$X^2$ für Wasserstoff, Fluor oder Chlor steht,

$R^3$ für Alkyl steht und

$R^4$ für Wasserstoff oder Alkyl steht,

indem man fluorierte Azomethine der Formel

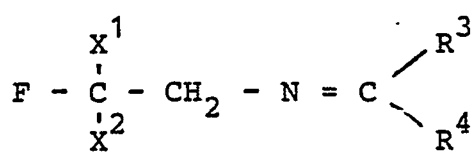

(VII)

in welcher

$X^1$, $X^2$, $R^3$ und $R^4$ die oben angegebene Bedeutung haben,

mit Wasserstoff unter einem Druck von 3 bis 15 bar in Gegenwart eines Katalysators, wie Platin auf Kohle,

Palladium auf Kohle oder Raney-Nickel, sowie in Gegenwart eines Verdünnungsmittels, z.B. eines Alkohols wie Methanol oder Ethanol, oder eines Ethers wie Dioxan, bei Temperaturen zwischen 0°C und 60°C, vorzugsweise zwischen 10°C und 50°C hydriert.

In der Formel (VI) steht $X^1$ vorzugsweise für Wasserstoff, Fluor oder Chlor. $X^2$ steht ebenfalls vorzugsweise für Wasserstoff, Fluor oder Chlor. $R^3$ steht vorzugsweise für Alkyl mit 1 bis 4 Kohlenstoffatomen, und $R^4$ steht vorzugsweise für Wasserstoff oder Alkyl mit 1 bis 4 Kohlenstoffatomen.

Die bei der Herstellung der fluorierten Amine der Formel (VI) nach dem oben beschriebenen Verfahren als Ausgangsstoffe benötigten Azomethine der Formel (VII) sind bisher noch nicht bekannt. Sie lassen sich jedoch herstellen, indem man Amine der Formel

$$F - \underset{X^2}{\overset{X^1}{C}} - CH_2 - NH_2 \qquad \text{(VIII)}$$

in welcher

$X^1$ und $X^2$ die oben angegebene Bedeutung haben,

mit Carbonylverbindungen der Formel

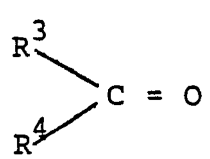

(IX)

in welcher

$R^3$ und $R^4$ die oben angegebene Bedeutung haben,

gegebenenfalls in Gegenwart eines Verdünnungsmittels, wie Pentan, Hexan, Cyclohexan, Chloroform, Tetrachlorkohlenstoff, Benzol, Toluol, Xylol, Chlorbenzol, Diethylether, Tetrahydrofuran oder Dioxan, bei Temperaturen zwischen -20°C und +60°C, vorzugsweise zwischen 0°C und +40°C umsetzt.

Die bei der Synthese der fluorierten Azomethine der Formel (VII) nach dem obigen Verfahren als Ausgangsstoffe benötigten Amine der Formel (VII) und Carbonylverbindungen der Formel (IX) sind bekannt oder lassen sich nach im Prinzip bekannten Methoden herstellen.

Als Verdünnungsmittel kommen bei der Umsetzung nach dem Verfahren (a) sowohl die als Reaktionskomponenten verwendeten fluorierten Amine der Formel (III) selbst, - sofern sie in flüssigem Zustand vorliegen oder bei niedrigen Temperaturen schmelzen - , als auch inerte organische Solventien in Frage. Hierzu gehören vorzugsweise chlorierte Kohlenwasserstoffe, wie Methylenchlorid und Chloroform, sowie Ether, wie Diethylether, Tetrahydrofuran und Dioxan. Ferner können auch Alkohole mit 1 bis 4 Kohlenstoffatomen, wie Methanol oder Isopropanol, oder auch Wasser als Verdünnungsmittel fungieren.

Als Katalysatoren können bei dem Verfahren (a) starke anorganische Basen, wie Natriumhydroxid oder Kaliumhydroxid, oder Amine, wie Triethylamin oder Triethylendiamin, verwendet werden.

Die Reaktionstemperaturen können bei dem Verfahren (a) innerhalb eines größeren Bereiches variiert werden. Im allgemeinen arbeitet man bei Temperaturen zwischen -50°C und +200°C, vorzugsweise zwischen 0°C und 100°C.

Bei der Durchführung des Verfahrens (a) setzt man auf 1 Mol an dimerem Keten der Formel (II) 2 Mol oder auch einen größeren Überschuß an fluoriertem Amin der Formel (III) sowie gegebenenfalls 0,01 bis 1, vorzugsweise 0,1 bis 0,5 Mol an Katalysator ein. Die Isolierung der entstehenden dihalogenierten Imidazol-carbonsäureamide der Formel (I) erfolgt nach üblichen Methoden. Im allgemeinen verfährt man in der Weise, daß man nach beendeter Umsetzung das Lösungsmittel und/oder die im Überschuß vorhandene Komponente (III) abdestilliert und den verbleibenden Rückstand mit wäßriger Alkalibase verrührt, dann ansäuert, das dabei kristallin anfallende Produkt abfiltriert und gegebenenfalls umkristallisiert.

Die bei dem erfindungsgemäßen Verfahren (b) als Ausgangsstoffe benötigten Imidazol-2-carbonsäure-amide sind durch die Formel (IV) allgemein definiert. In dieser Formel haben $R^1$ und $R^2$ vorzugsweise diejenigen

Bedeutungen, die bereits im Zusammenhang mit der Beschreibung der erfindungsgemäßen Stoffe der Formel (I) vorzugsweise für diese Reste genannt wurden.

Die Imidazol-2-carbonsäure-amide der Formel (IV) sind bisher noch nicht bekannt; sie lassen sich jedoch nach üblichen Methoden herstellen. So erhält man diese Stoffe, indem man das dimere Keten der Formel

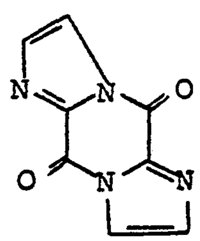

(X)

mit fluorierten Aminen der Formel

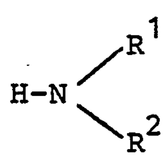

(III)

in welcher

$R^1$ und $R^2$ die oben angegebene Bedeutung haben,

gegebenenfalls in Gegenwart eines Verdünnungsmittels sowie gegebenenfalls in Gegenwart eines Katalysators bei Temperaturen zwischen -50°C und +200°C, vorzugsweise zwischen 0°C und +100°C, umsetzt.

Das bei der Herstellung der Imidazol-2-carbonsäure-amide der Formel (IV) als Ausgangsstoff benötigte dimere Keten der Formel (X) ist bisher noch nicht beschrieben worden. Es läßt sich jedoch herstellen, indem man Imidazol-2-carbonsäure mit einem Überschuß an Thionylchlorid gegebenenfalls in Gegenwart von Dimethylformamid als Katalysator unter Rückfluß erhitzt.

Die Imidazol-2-carbonsäure ist bekannt (vgl. Acta Chem. Scand. 21, 279 (1967)).

Bei der Herstellung der Imidazol-2-carbonsäure-amide der Formel (IV) nach dem oben angegebenen Verfahren kommen als Verdünnungsmittel bzw. als Katalysatoren alle diejenigen Stoffe in Betracht, die in diesem Zusammenhang bei der Beschreibung des erfindungsgemäßen Verfahrens (a) erwähnt wurden. Die Durchführung dieses Verfahrens erfolgt so wie es bei dem erfindungsgemäßen Verfahren (a) beschrieben wurde. Bei der Aufarbeitung verfährt man nach üblichen Methoden. Im allgemeinen geht man so vor, daß man nach beendeter Umsetzung das Lösungsmittel und/oder die im Überschuß vorhandene Komponente der Formel (III) abdestilliert und den verbleibenden Rückstand mit wäßriger Salzsäure verrührt, dann mit wäßriger Alkalibase alkalisch macht, das dabei kristallin anfallende Produkt abtrennt und gegebenenfalls umkristallisiert.

Als Chlorierungs- und Bromierungsmittel können bei dem erfindungsgemäßen Verfahren (b) die Halogene Chlor

und Brom, aber auch Chlor abgebende Substanzen, wie Sulfurylchlorid, verwendet werden.

Als Säurebindemittel kommen bei dem erfindungsgemäßen Verfahren (b) vorzugsweise Alkali- oder Erdalkalihydroxide, -carbonate oder -hydrogencarbonate, wie z.B. Natriumhydroxid oder Natriumhydrogencarbonat, in Frage.

Als Verdünnungsmittel kommen bei dem erfindungsgemäßen Verfahren (b) alle unter den Reaktionsbedingungen inerten Lösungsmittel in Betracht. Hierzu gehören vorzugsweise Methylenchlorid und Chloroform. Es kann jedoch auch in wäßrigem Medium gegebenenfalls in Gegenwart eines Säurefängers gearbeitet werden.

Die Reaktionstemperaturen können bei dem erfindungsgemäßen Verfahren (b) innerhalb eines größeren Bereiches variiert werden. Im allgemeinen arbeitet man bei Temperaturen zwischen -50°C und +150°C, vorzugsweise zwischen -20°C und +100°C. Dient Wasser als Verdünnungsmittel, so arbeitet man zweckmäßigerweise zwischen 0°C und +50°C.

Bei der Durchführung des erfindungsgemäßen Verfahrens (b) setzt man auf 1 Mol an Imidazol-2-carbonsäure-amid der Formel (IV) 2 Mol oder auch einen größeren Überschuß, vorzugsweise 2,5 Mol an Chlorierungs- bzw. Bromierungsmittel, sowie bei Verwendung von Wasser als

Verdünnungsmittel 2 oder mehr Äquivalente eines Säurebinders ein. Vorzugsweise verfährt man so, daß man das Imidazol-2-carbonsäure-amid der Formel (IV) in einem Verdünnungsmittel vorlegt, dann das Halogenierungsmittel, gegebenenfalls unter Kühlung, in dem Maße zudosiert, daß die gewählte Reaktionstemperatur eingehalten wird. Zur Vervollständigung der Reaktion kann das Reaktionsgemisch nach beendeter Zugabe des Halogenierungsmittels noch weitere 1 bis 5 Stunden auf der gewählten Temperatur gehalten werden. Im Falle der Verwendung von Wasser als Verdünnungsmittel kann der Säurefänger entweder mit dem Imidazol-2-carbonsäure-amid der Formel (IV) vorgelegt werden oder aber nach der Zugabe des Halogenierungsmittels zugefügt werden. Auch die gleichzeitige Zugabe von Säurefänger und Halogenierungsmittel ist möglich. In einer weiteren Variante kann auch das Halogenierungsmittel vorgelegt werden und das Imidazol-2-carbonsäure-amid der Formel (IV) in Lösung hinzugegeben werden. Die Isolierung der Reaktionsprodukte erfolgt nach üblichen Methoden. Im allgemeinen verfährt man so, daß man bei Verwendung von inerten organischen Solventien als Verdünnungsmittel letztere nach beendeter Umsetzung abzieht und den Rückstand gegebenenfalls reinigt, indem man das Rohprodukt mit wäßriger Salzsäure im Verhältnis 1:1 bei 20°C behandelt. Dabei gehen monohalogenierte Verbindungen und nicht halogeniertes Ausgangsprodukt in Lösung, während das dihalogenierte Produkt in fester Form verbleibt und abfiltriert werden kann. Arbeitet

man in Gegenwart von Wasser als Verdünnungsmittel, so erfolgt die Aufarbeitung im allgemeinen dadurch, daß man das Reaktionsgemisch durch Zugabe von wäßriger Alkalibase alkalisch macht, das ausfallende Produkt abtrennt, wäscht und gegebenenfalls umkristallisiert.

Das bei dem Verfahren (c) als Ausgangsprodukt benötigte Perchlordiazafulven der Formel (V) ist bereits bekannt (vgl. DE-A 24 54 326).

Als Verdünnungsmittel kommen bei der Umsetzung nach dem Verfahren (c) sowohl die als Reaktionskomponenten verwendeten fluorierten Amine der Formel (III) selbst, - sofern sie in flüssigem Zustand vorliegen oder bei niedrigen Temperaturen schmelzen - , als auch inerte organische Solventien in Frage. Hierzu gehören vorzugsweise Ether wie Dioxan oder Tetrahydrofuran oder aliphatische Nitrile wie Acetonitril. Man kann aber auch Wasser verwenden.

Als Säurebindemittel können bei dem erfindungsgemäßen Verfahren (c) alle üblichen Säureakzeptoren verwendet werden. Vorzugsweise in Frage kommen Alkali- oder Erdalkalihydroxide, -carbonate oder -hydrogencarbonate, wie z.B. Natriumhydroxid oder Natriumhydrogencarbonat.

Die Reaktionstemperaturen können bei dem erfindungsgemäßen Verfahren (c) innerhalb eines größeren Bereiches variiert werden. Im allgemeinen arbeitet man bei Temperaturen zwischen 0°C und 150°C, vorzugsweise zwischen 20°C und 100°C.

Bei der Durchführung des erfindungsgemäßen Verfahrens (c) setzt man auf 1 Mol an Perchlordiazafulven der Formel (V) 1 Mol oder auch einen größeren Überschuß an fluoriertem Amin der Formel (III) sowie gegebenenfalls 2 Mol oder auch einen größeren Überschuß an Säurebindemittel ein. Die Aufarbeitung erfolgt nach üblichen Methoden. Im allgemeinen verfährt man in der Weise, daß man nach beendeter Umsetzung das Verdünnungsmittel und/oder die im Überschuß vorhandene Komponente der Formel (III) abdestilliert, den verbleibenden Rückstand mit Wasser verrührt, das dabei kristallin anfallende Produkt abtrennt und gegebenenfalls durch Umkristallisation oder Sublimation reinigt.

Die erfindungsgemäßen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dicotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe können als solche oder in ihren Formulierungen auch in Mischung mit bekannten Herbiziden zur Unkrautbekämpfung Verwendung finden, wobei Fertigformulierung oder Tankmischung möglich ist.

Die erfindungsgemäßen Stoffe eignen sich insbesondere zur selektiven Unkrautbekämpfung in Mais und Getreide (z.B. Weizen und Hafer).

Die Wirkstoffe können in die üblichen Formulierungen übergeführt werden, wie Lösungen, Emulsionen, Suspensionen, Pulver, Schäume, Pasten, Granulate, Wirkstoffimprägnierte Natur- und synthetische Stoffe und Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

Z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäß verwendbaren Wirkstoffe können als solche oder in ihren Formulierungen auch in Mischung mit bekannten Herbiziden zur Unkraubekämpfung Verwendung finden, wobei Fertigformulierung oder Tankmischung möglich ist. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die Wirkstoffe können als solche, in Form ihrer Formulierungen oder der daraus durch weiteres Verdünnen bereiteten Anwendungsformen, wie gebrauchsfertige Lösungen, Suspensionen, Emulsionen, Pulver und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die aufgewandte Wirkstoffmenge kann in größeren Bereichen schwanken. Sie hängt im wesentlichen von der Art des gewünschten Effekts ab. Im allgemeinen liegen die Aufwandmengen zwischen 0,05 und 10 kg Wirkstoff pro ha, vorzugsweise zwischen 0,1 und 5 kg/ha.

Die Herstellung und die Verwendung der erfindungsgemäßen Stoffe geht aus den nachfolgenden Beispielen hervor.

Herstellungsbeispiele:

Beispiel 1

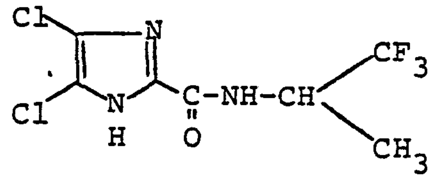

12,39 g (0,11 Mol) 2-Amino-1,1,1-trifluorpropan in 100 ml Dioxan wurden bei Raumtemperatur unter Kühlung tropfenweise mit einer Lösung von 21,6 g (0,099 Mol) Perchlordiazafulven der Formel

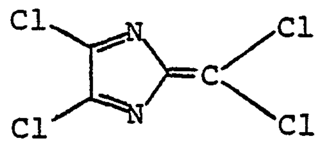

in 50 ml Dioxan versetzt. Nach zweistündigem Erhitzen unter Rückfluß wurde abgekühlt, im Wasserstrahlvakuum zur Trockne einrotiert, mit Wasser verrührt und abgesaugt. Nach dem Waschen mit Wasser und Trocknen erhielt man 24,8 g (90,7 % der Theorie) 4,5-Dichlor-imidazol-2-carbonsäure-N-(2,2,2-trifluor-isopropyl)-amid. Schmelzpunkt 160°C (aus Hexan). Die Verbindung ist bei 125°C/0,05 Torr sublimierbar.

Nach der im Beispiel 1 angegebenen Methode werden auch die in der folgenden Tabelle formelmäßig angegebenen Stoffe hergestellt:

Tabelle 1

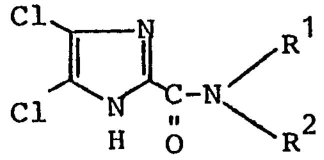

(Ia)

| Beispiel-Nr. | $R^1$ | $R^2$ | Schmelzpunkt in °C | umkristallisiert aus |
|---|---|---|---|---|
| 2 | $-CH_2-CF_2-CH_3$ | H | 204 | wenig Acetonitril |
| 3 | $-CH_2-CF_3$ | H | 180 | Acetonitril |
| 4 | $-CH_2-CF_3$ | $-CH_2-CH_3$ | 122 | Petrolether |
| 5 | $-CH_2-CH_2-CCl_2F$ | H | 150 | Cyclohexan |

Beispiel 6

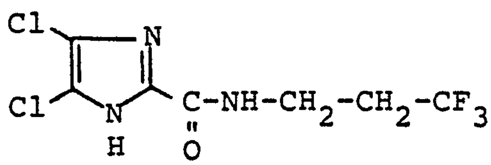

Eine Suspension von 13,6 g (0,042 Mol) des dimeren Ketens der Formel

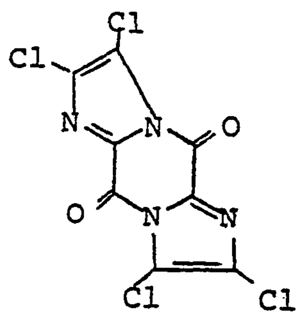

in 130 ml Dioxan wurde bei Raumtemperatur mit 11,2 g (0,1 Mol) Triethylendiamin der Formel

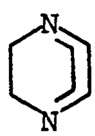

und dann mit 11,3 g (0,1 Mol) 1-Amino-3,3,3-trifluorpropan versetzt. Nach einstündigem Erhitzen unter Rückfluß wurde abgekühlt, im Wasserstrahlvakuum zur Trockne einrotiert, der Rückstand mit 100 ml 20 %iger wäßriger Natronlauge verrührt und dann mit Salzsäure auf pH = 1 angesäuert. Nach dem Absaugen, Waschen mit Wasser und Trocknen erhielt man 19,0 g (entsprechend 81,9 % der Theorie) 4,5-Dichlorimidazol-2-carbonsäure-N-(3,3,3-trifluor-n-propyl)-amid. Schmelzpunkt 177°C (aus Acetonitril).

Beispiel 7

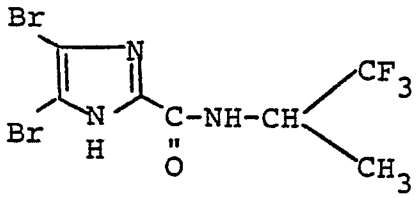

Eine Suspension von 14,5 g (0,07 Mol) Imidazol-2-carbonsäure-N-(2,2,2-trifluor-isopropyl)-amid in 150 ml Wasser wurde bei 0 - 10°C unter Eiskühlung innerhalb etwa einer Stunde mit 25,6 g (0,16 Mol) Brom tropfenweise versetzt. Nach dreistündigem Nachrühren wurde durch Zutropfen von wäßriger 1n-Natronlauge auf pH = 6 eingestellt, abgesaugt, mit Wasser gewaschen und getrocknet. Man erhielt 16,0 g (entsprechend 62 % der Theorie) 4,5-Dibromimidazol-2-carbonsäure-N-(2,2,2-trifluor-isopropyl)-amid. Nach dem Umkristallisieren aus wenig Acetonitril erhielt man feine Nadeln vom Schmelzpunkt 185°C.

Herstellung von Ausgangsprodukten:

Beispiel III-1

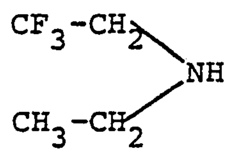

a) 125 g (1 Mol) Ethyliden-2,2,2-trifluorethylamin werden in 250 ml absolutem Ethanol gelöst, mit 4 g Platin auf Kohle (5 %ig) versetzt und bei 30°C unter einem Wasserstoffdruck von 10 bar 90 Minuten lang hydriert. Nach dem Abfiltrieren des Katalysators wird mit konzentrierter Salzsäure angesäuert und unter vermindertem Druck bis zur Trockne eingeengt. Das dabei anfallende Produkt wird mit 100 ml 50 %iger wäßriger Natronlauge versetzt und destilliert. Man erhält auf diese Weise 83 g (65 % der Theorie) an 2,2,2-Trifluor-ethyl-N-ethylamin.

Kp = 61 - 62°C

$n_D^{20}$ = 1,3335

b) Herstellung des Vorproduktes der Formel

$$CF_3 - CH_2 - N = CH - CH_3 \qquad (VII-1)$$

Zu 99 g (1 Mol) 2,2,2-Trifluorethylamin werden innerhalb von 60 Minuten unter Eiskühlung 44 g (1 Mol) frisch destillierter Acetaldehyd zugetropft.

Man läßt 1 Stunde nachrühren, gibt 15 g festes Kaliumhydroxid hinzu und trennt die Phasen. Die organische Phase wird über etwa 5 g festem Kaliumhydroxid getrocknet und anschließend über eine Kolonne destilliert. Nach einem Vorlauf (20 g; Kp: 18 - 42°C), der aus nicht umgesetztem Amin und Acetaldehyd besteht, erhält man 77,5 g (62 % der Theorie) an Ethyliden-2,2,2-trifluorethyl-amin.

Kp = 73 - 74°C
$n_D^{20}$ = 1,3415

Nach der im Beispiel III-1 unter (a) angegebenen Methode werden auch die in den folgenden Beispielen formelmäßig aufgeführten fluorierten Amine der Formel (III) erhalten.

Beispiel III-2

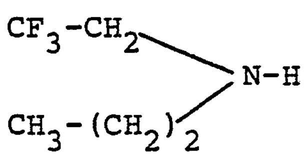

Ausbeute: 66 % der Theorie
$n_D^{20}$ = 1,3462
Kp = 74°C

Beispiel III-3

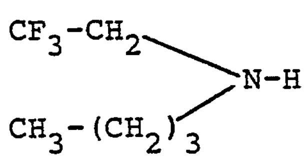

Ausbeute: 72 % der Theorie
$n_D^{20}$ = 1,3590
Kp = 84°C

Nach der im Beispiel III-1 unter (b) angegebenen Methode werden auch die in den folgenden Beispielen formelmäßig aufgeführten fluorierten Azomethine der Formel (VII) erhalten.

Beispiel VII-2

$$CF_3 - CH_2 - N = CH - CH_2 - CH_3$$

Ausbeute: 64 % der Theorie
Kp = 93 - 95°C

Beispiel VII-3

$$CF_3 - CH_2 - N = CH_2 - CH_2 - CH_2 - CH_3$$

Ausbeute: 72 % der Theorie
Kp = 116 – 118°C.

Beispiel IV-1

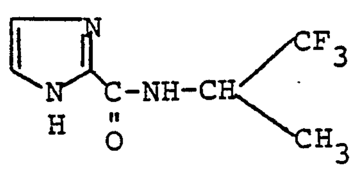

a) In eine Mischung aus 24,37 g (0,22 Mol) 2-Amino-1,1,1-trifluorpropan und 200 ml Wasser wurden bei Raumtemperatur 18,8 g (0,1 Mol) dimeres Keten der Formel

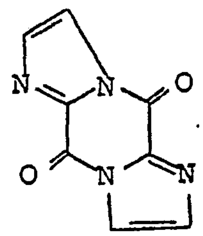

eingerührt. Nach vierstündigem Nachrühren bei Raumtemperatur war die gelbe Farbe des dimeren Ketens verschwunden. Anschließend wurde im Vakuum einrotiert, der Rückstand in 500 ml 1n-Salzsäure verrührt, von geringfügigen Verunreinigungen abfiltriert und mit wäßriger Natronlauge auf einen pH-Wert von 6 gebracht. Nach dem Absaugen, Waschen mit Wasser und Trocknen erhielt man 22,15 g (54 % der Theorie) Imidazol-2-carbonsäure-N-(2,2,2-trifluor-isopropyl)-amid vom Schmelzpunkt 234°C. Das Produkt kann aus Acetonitril umkristallisiert werden.

b) Herstellung des Vorproduktes

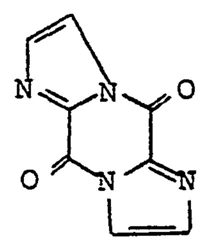

(X)

Variante α

11,2 g (0,1 Mol) Imidazol-2-carbonsäure wurden mit 100 ml Thionylchlorid 5 Stunden unter Rückfluß gerührt. Nach dem Erkalten wurde abgesaugt, mit etwas Petrolether gewaschen und getrocknet. Man erhielt so in fast quantitativer Ausbeute das 5H,10H-diimid-

azo/1,2-a:1',2'-d/pyrazin-5,10-dion in Form eines gelben Pulvers. Die Substanz schmilzt noch nicht bei 290°C. Sie ist bei 200 - 250°C/0,01 Torr unzersetzt in gelben Kristallen sublimierbar.

IR (KBr): 3137, 1735, 1522, 1445, 1387, 1331, 1274, 1161, 1059, 1018, 810, 800, 748, 699, 651 $cm^{-1}$.

Variante ß

Man verfuhr wie unter α) mit dem Unterschied, daß man 1 ml Dimethylformamid zusetzte. Die Umsetzung zur Verbindung der Formel

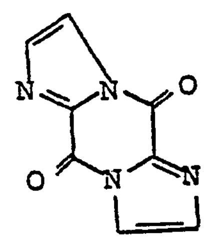

(X)

war, wie der Vergleich der IR-Spektren ergab, bereits nach etwa einer halben Stunde praktisch vollständig.

In den folgenden Verwendungsbeispielen wurde die nachstehend formelmäßig angegebene Substanz als Vergleichspräparat eingesetzt:

A = 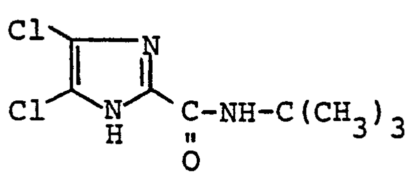 (bekannt)

(4,5-Dichlorimidazol-2-carbonsäure-tert.-butylamid)

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigen die erfindungsgemäßen Wirkstoffe (3), (4) und (6) eine bessere Wirksamkeit als die Vergleichssubstanz (A).

Beispiel B

Post-emergence-Test / Gewächshaus

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschte Wirkstoffmenge pro Flächeneinheit ausgebracht wird. Die Konzentration der Spritzbrühe wird so gewählt, daß in 2000 l Wasser/ha die jeweils gewünschte Wirkstoffmenge ausgebracht wird. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigen die erfindungsgemäßen Verbindungen (3) und (6) eine bessere Wirksamkeit als die Vergleichssubstanz (A).

Formulierungsbeispiel

Zur Herstellung eines Spritzpulvers werden

| | | |
|---|---|---|
| 70 | Gewichtsteile | Wirkstoff gemäß Beispiel 1 |
| 2 | Gewichtsteile | Netzmittel |
| 3 | Gewichtsteile | Dispergiermittel auf Basis von Ligninsulfonat |
| 5 | Gewichtsteile | Alkylarylsulfonat |
| 5 | Gewichtsteile | hochdisperse Kieselsäure |
| 15 | Gewichtsteile | Kaolin |

in einem Lödige-Mischer intensiv gemischt und anschließend in einem Mikronizer 8" fein gemahlen. Das erhaltene Pulver wird vor der Ausbringung auf die Pflanzen in der jeweils gewünschten Menge mit Wasser vermischt.

## Patentansprüche

1. Dihalogenierte Imidazolcarbonsäure-amide der Formel

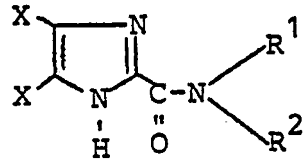

(I)

in welcher

X für Chlor oder Brom steht,

$R^1$ für Fluoralkyl oder Fluorchloralkyl steht und

$R^2$ für Wasserstoff oder Alkyl steht.

2. Dihalogenierte Imidazolcarbonsäure-amide der Formel (I), in welcher X für Chlor oder Brom steht, $R^1$ für Fluoralkyl mit 1 bis 8 Kohlenstoffatomen und 1 bis 9 Fluoratomen oder für Fluorchloralkyl mit 1 bis 8 Kohlenstoffatomen und bis zu 9 Fluor- und Chloratomen steht und $R^2$ für Wasserstoff oder Alkyl mit 1 bis 6 Kohlenstoffatomen steht.

3. Verfahren zur Herstellung von dihalogenierten Imidazolcarbonsäure-amiden der Formel

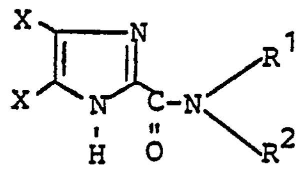

(I)

in welcher

X für Chlor oder Brom steht,

$R^1$ für Fluoralkyl oder Fluorchloralkyl steht und

$R^2$ für Wasserstoff oder Alkyl steht,

dadurch gekennzeichnet, daß man

a) das dimere Keten der Formel

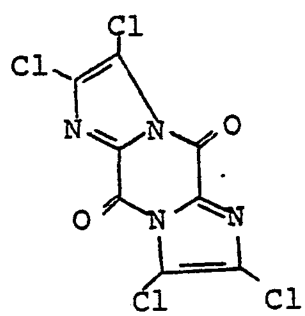

(II)

mit fluorierten Aminen der Formel

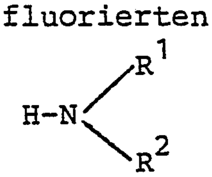

(III)

in welcher

$R^1$ und $R^2$ die oben angegebene Bedeutung haben,

gegebenenfalls in Gegenwart eines Verdünnungsmittels sowie gegebenenfalls in Gegenwart eines Katalysators umsetzt,

oder

b) Imidazol-2-carbonsäure-amide der Formel

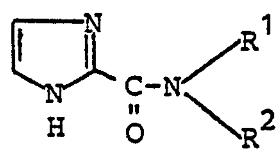

(IV)

in welcher

$R^1$ und $R^2$ die oben angegebene Bedeutung haben,

mit mindestens der zur Dihalogenierung stöchiometrisch erforderlichen Menge an Chlorierungs- oder Bromierungsmittel in Gegenwart eines unter den Reaktionsbedingungen inerten Verdünnungsmittels sowie gegebenenfalls in Gegenwart eines säurebindenden Mittels umsetzt,

oder

c) Perchlordiazafulven der Formel

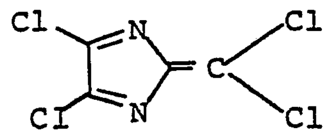

(V)

mit fluorierten Aminen der Formel

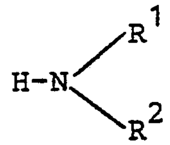

(III)

in welcher

$R^1$ und $R^2$ die oben angegebene Bedeutung haben,

in Gegenart eines Verdünnungsmittels sowie gegebenenfalls in Gegenwart eines Säurebindemittels umsetzt und anschließend mit Wasser hydrolysiert.

4. Herbizide Mittel, gekennzeichnet durch einen Gehalt an mindestens einem dihalogenierten Imidazolcarbonsäure-amid der Formel (I).

5. Verfahren zur Bekämpfung von Unkräutern, dadurch gekennzeichnet, daß man dihalogenierte Imidazolcarbonsäure-amide der Formel (I) auf Unkräuter und/oder deren Lebensraum ausbringt.

6. Verwendung von dihalogenierten Imidazol-carbonsäure-amiden der Formel (I) zur Bekämpfung von Unkräutern.

7. Verfahren zur Herstellung von herbiziden Mitteln, dadurch gekennzeichnet, daß man dihalogenierte Imidazol-carbonsäure-amide der Formel (I) mit Streckmitteln und/oder oberflächenaktiven Stoffen vermischt.

8. Dihalogeniertes Imidazolcarbonsäureamid der Formel

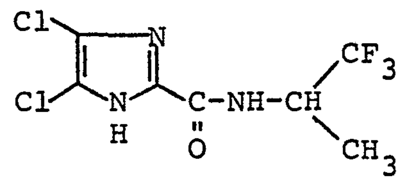

9. Dihalogeniertes Imidazolcarbonsäureamid der Formel

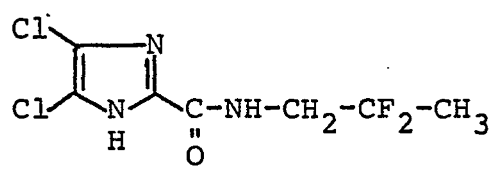

10. Dihalogeniertes Imidazolcarbonsäureamid der Formel